(19) Europäisches Patentamt
European Patent Office
Office européen des brevets

(11) EP 3 659 577 A1

# (12) EUROPEAN PATENT APPLICATION

(43) Date of publication:
**03.06.2020 Bulletin 2020/23**

(21) Application number: **18209099.3**

(22) Date of filing: **29.11.2018**

(51) Int Cl.:
***A61K 8/365*** *(2006.01)* ***A61Q 5/06*** *(2006.01)*
***A61K 8/898*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Kao Germany GmbH**
**64297 Darmstadt (DE)**

(72) Inventors:
- **Picker, Andreas**
  **64297 Darmstadt (DE)**
- **Klawitter, Iris**
  **64297 Darmstadt (DE)**

## (54) KERATIN FIBER STRAIGHTENING COMPOSITION, PROCESS, AND KIT-OF-PARTS

(57) The present invention is directed to a keratin fibers straightening composition, process, and kit-of-parts. It has been unexpectedly discovered that a composition comprising glyoxylic acids and/or its variants and Polysilicone-29 showed superior keratin fibers straightening effect while maintaining the fiber's cosmetic properties.

EP 3 659 577 A1

## Description

### Field of the invention

The present invention relates to the field of straightening of keratin fibers, preferably human keratin fibers, more preferably human hair. Furthermore, a process for straightening the keratin fibers as well as a kit-of-parts is disclosed.

### Background of the invention

Changing ones hair shape has always been en vogue. Already in the ancient Egyptian era, long, straight hair was popular. Women, who were not blessed with this kind of natural hair used fire heated iron plates to transform their hair into the desired shape. This procedure often led to severe burns of the face and hands. It was not before the early $19^{th}$ century that straightening irons became more professional with the invention of a hair tong made by Marcel Grateau. Nowadays, hot tools such as curling or straightening irons are more popular than ever before. They got into the focus of consumers in both salon and mass market. These tools lead to satisfying results as they work at very high temperatures up to 230°C. When dry hair is getting in contact with the hot iron and a proper pressure is applied at the same time, the hair shape can be fixed for a certain amount of time until humidity returns it to the original state. Unfortunately, application of such hot temperature leads to hair damage and thus a bad hair feel after use.

The prior art has primarily tackled the durability problem while making non-reductive processes for straightening of hair available. WO2011104282 and WO2012010351 disclose hair-straightening processes employing glyoxylic acid treatments and combining them with a heating step. This procedure leads to good straightening results, but comes with a few disadvantages. Due to the high concentration of glyoxylic acid that is needed for straightening very curly hair, there always is a risk of irritating scalp or skin. Furthermore, the treatment has an undesired effect on the client's natural hair color, often leading to a color shift and/or undesired lightening. Therefore, it is not uncommon to combine the straightening routine with an oxidative coloring routine as disclosed in WO2014067702.

In FR2997845 and FR3044903 hair treatment compositions and processes are disclosed employing acids and monomeric silanes in combination with a heating step. In FR2997847 a combination of glyoxylic acid with aminosilicones and a heating step is disclosed. However, the aforementioned prior art is silent on Polysilicone-29.

Despite the attempts of the prior art, there is a huge need for products which allow for a more effective straightening of keratin fibers while reducing hair damage, leaving a pleasant hair feel after application and enhancing hair shine, and do not have a detrimental effect on the natural hair color of the customer.

So far the prior art has not fully achieved the aforementioned objectives.

### Summary of the invention

Inventors of the present invention have unexpectedly found that a combination of glyoxylic acid and/or its various forms with Polysilicone-29 provided a superior hair straightening effect in comparison to each of the components used alone while maintaining a healthy state of the hair resulting in very good cosmetic feel of the treated keratin fibers. In addition it has been found that there is no visually perceivable color shift of the keratin fibers after carrying out the treatment.

Therefore, the first object of the present invention is a composition for straightening of keratin fibers, preferably human keratin fibers, more preferably human hair, having a pH of 4 or less and comprising

- glyoxylic acid and/or its hydrate(s) and/or its salt(s), and
- Polysilicone-29.

The second object of the present invention is a two-part composition comprising the parts A and B, which are provided for separate storage and for mixing directly prior to the application onto the keratin fibers,
wherein part A comprises glyoxylic acid and/or its hydrate(s) and/or its salt(s) having a pH of less than 4, and part B comprises Polysilicone-29 having a pH in the range of 2 to 10.

The third object of the present invention is a process for semi-permanent straightening of keratin fibers, preferably human keratin fibers, more preferably human hair characterized in that it comprises the steps of:

a) optionally washing the keratin fibers and/or drying the keratin fibers,

b) applying the composition and/or two-part composition as defined above onto keratin fibers,

c) leaving the composition onto the keratin fibers for 1 min to 120 min, preferably for a time period in the range of

2 min to 60 min, more preferably in the range of 2 min to 30 min, further more preferably in the range of 2 min to 10 min,

d) optionally rinsing-off the keratin fibers,

e) drying the keratin fibers,

f) treating the keratin fibers with a straightening iron having a surface temperature of 180°C ± 50°C, preferably in the range of 170°C to 200°C,

g) optionally rinsing-off and/or washing the keratin fibers and/or drying the keratin fibers.

The time range for leaving the composition on the keratin fibers in step c) from 1 to 10 min is particularly preferred for home application of the composition, whereas the longer process times are preferred for salon application.
It is preferred that the weight ratio of keratin fibers to composition in step b) is in the range of 0.5:2 to 2:0.5.
It is also preferred that the steps a) to g) are repeated with a time period of 8 h and 72 h between each full process run from above.
A further object of the present invention is a process for semi-permanent straightening of keratin fibers, preferably human keratin fibers, more preferably human hair characterized in that it comprises the steps of:

a) optionally washing the keratin fibers and/or drying the keratin fibers,

b) applying a composition having a pH of 4 or less and comprising glyoxylic acid and/or its hydrate(s) and/or its salt(s),

c) leaving the composition onto the keratin fibers for 1 min to 120 min, preferably for a time period in the range of 2 min to 60 min, more preferably in the range of 2 min to 30 min, further more preferably in the range of 2 min to 10 min,

d) rinsing-off the keratin fibers,

e) applying a composition having a pH in the range of 2 to 10 and comprising Polysilicone-29,

f) optionally rinsing-off the keratin fibers,

g) drying the keratin fibers,

h) treating the keratin fibers with a straightening iron having a surface temperature of 180°C ± 50°C, preferably in the range of 170°C to 200°C,

i) optionally rinsing-off and/or washing the keratin fibers and/or drying the keratin fibers.

The time range for leaving the composition on the keratin fibers in step c) from 1 to 10 min is particularly preferred for home application of the composition, whereas the longer process times are preferred for salon application.
It is suitable that the steps a) to g) are repeated with a time period of 8 h and 72 h between each full process run from above.
It is preferred that the weight ratio of keratin fibers to compositions in steps b) and e) is in the range of 0.5:2 to 2:0.5.
Still a further object of the present invention is a kit-of-parts comprising the composition and/or two-part composition as defined above and a straightening tool for keratin fibers, preferably a hair straightening iron.

**Detailed description of the invention**

**Glyoxylic acid**

The composition comprises glyoxylic acid and/or its hydrate(s) and/or its salt(s) at a total concentration is in the range of 0.1 % to 20% by weight, preferably in the range of 0.2% to 10% by weight, more preferably in the range of 0.25% to 5% by weight, further more preferably in the range of 0.35% to 5% by weight, calculated to the total of the composition. The concentration ranges between 5% and 20% by weight of glyoxylic acid and/or its hydrate(s) and/or its salt(s) is particularly suitable for hair salon application performed by a professional, whereas the 0.1% to 5% by weight concentration of the compounds are suitable for consumer products for home use.
Suitable concentration ranges are illustrated in examples 3-7.

## Polysilicone-29

Polysilicone-29 is the INCI name of a polymeric silane offered for sale by Momentive Performance Materials Inc.

For aspects of the present invention where the polymeric silane and glyoxylic acid and/or its hydrates and/or its salt(s) are within the same one-part composition, the pH is less than 7, preferably 4 or less, more preferably it is in the range of pH 1 to 3.5. Suitable pH ranges are illustrated in example 3.

In another aspect of the present invention, Polysilicone-29 is kept separate from the composition comprising glyoxylic acid and/or its hydrate(s) and/or its salt(s) and the compositions comprising the compounds are mixed directly prior to their use. Having a mixing step prior to application onto keratin fibers for this embodiment allows for greater flexibility with mixing ratios of the two compositions.

The latter aspect is then available as a two-part composition in line with the second object of the present invention. This aspect is illustrated in example 8.

For this aspect, the pH of the composition comprising glyoxylic acid and/or its salt(s) and/or its hydrate(s) is less than 7, preferably 4 or less, more preferably it is in the range of pH 1 to 3.5. For the composition part comprising Polysilicone-29, the pH may be in the range of 2 to 10. However, after mixing both compositions, the resulting pH must be in the range of less than 7, preferably 4 or less, more preferably it is in the range of pH 1 to 3.5 to make full use of the glyoxylic acid straightening potential.

In another aspect of the present invention, Polysilicone-29 is kept separate from the composition comprising glyoxylic acid and/or its hydrate(s) and/or its salt(s) and is also applied subsequently onto keratin fibers. According to this embodiment, the composition comprising glyoxylic acid and/or its hydrate(s) and/or its salt(s) is applied first, and then the composition comprising Polysilicone-29 is applied onto keratin fibers without a rinsing step between applying the compositions. This embodiment is illustrated in examples 2, 6, and 7. The pH ranges are similar as disclosed for the two-part composition.

The skilled reader notices that with the absence of the rinsing step the compositions comprising Polysilicone-29 and glyoxylic acid is formed on the keratin fibers.

In yet another aspect of the present invention, the Polysilicone-29 is kept separate from the composition comprising glyoxylic acid and/or its hydrate(s) and/or its salt(s) and also applied subsequently on the keratin fibers. According to this embodiment, the composition comprising glyoxylic acid and/or its hydrate(s) and/or its salt(s) is applied first, and then the composition comprising Polysilicone-29 is applied onto keratin fibers with a rinsing step between applying the compositions. The pH ranges are similar as disclosed for the two-part composition.

Irrespective of the aspects listed above, the composition comprising Polysilicone-29 comprises this compound at a total concentration in the range of 0.01 % to 10% by weight, preferably in the range of 0.02% to 8% by weight, more preferably in the range of 0.05% to 6% by weight, further more preferably in the range of 0.1% to 2% by weight, calculated to the total of the composition.

Also irrespective of the aspects listed above, the weight ratio of glyoxylic acid and/or its hydrate(s) and/or its salt(s) to Polysilicone-29 applied onto keratin fibers is in the range of 10 to 0.1. This weight ratio applies to the embodiments where glyoxylic acid and/or its hydrate(s) and/or its salt(s) and Polysilicone-29 are present in one composition, but also to the embodiments where both compounds are kept separate prior to their application onto keratin fibers.

## Cosmetic forms of composition

Suitable cosmetic forms of the compositions of the present inventions are solutions, dispersions, thickened gels and/or emulsions, as well as foams.

## Thickening polymers

For preparation of a thickened gel, the composition comprises a thickening agent, preferably a thickening polymer, more preferably an associative and/or non-associative thickening polymer.

It is to be noted that the thickening polymer must have the property of thickening the composition under the low pH conditions, namely at pH of 4 or less. Thus, suitable thickeners provide for a solution/gel having a viscosity of at least 500 mPas at 25°C, measured with a Brookfield viscometer and spindle #5, at pH of 4 or less. More preferably suitable thickening polymers are also resistant to partial neutralization of glyoxylic acid and/or its hydrate(s) and/or its salt(s).

Suitable thickening polymers are carbohydrate-based non-associative thickening polymers, still further more preferably they are selected from xanthan gum and/or dehydroxanthan gum, and hydroxyethylcellulose.

The concentration of thickening agents in the composition of the present invention is in the range from 0.1% to 5% by weight, preferably 0.2% to 3% by weight, calculated to the total of the composition.

The gel form of the compositions of the present invention is illustrated in examples 4, 5, 7, and 8.

It is to be noted that such thickened gels may comprise one or more hydrophobic compound(s) at a concentration below 2% by weight, calculated to the total of the composition. Such a viewpoint is illustrated in example 4.

**Hydrophobic compounds**

In case the composition of the present invention presents itself as an emulsion or a gel, the composition comprises one or more hydrophobic compound(s), preferably selected from $C_{12}$ to $C_{22}$ fatty alcohols, fatty acid esters having a $C_{12}$ to $C_{22}$ fatty acid esterified with a linear or branched, saturated or unsaturated $C_3$ to $C_{18}$ fatty alcohol, natural and/or vegetable oils, hydrocarbon-based compounds, and/or their mixtures.

Suitable $C_{12}$ to $C_{22}$ fatty alcohols are lauryl alcohol, tridecyl alcohol, myristyl alcohol, pentadecyl alcohol, cetyl alcohol, palmitoleyl alcohol, heptadecyl alcohol, stearyl alcohol, nonadecyl alcohol, arachidyl alcohol, behenyl alcohol, and/or their mixtures, in particular cetearyl alcohol.

Suitable fatty acid esters consisting of linear or branched, saturated or unsaturated fatty acids with $C_{12}$ to $C_{22}$ being esterified with linear or branched primary alcohols with $C_3$ to $C_{18}$ are octyl palmitate, isocetyl palmitate, isopropyl palmitate, octyl stearate, oleyl oleate, and myristyl myristate, as well as their mixtures.

Suitable petrolatum-based compounds are liquid paraffins, especially paraffinum perliquidum and paraffinum subliquidum, and mineral oil, in particular white mineral oil.

Suitable natural and/or vegetable oils are olive oil, almond oil, avocado oil, wheatgerm oil, and castor oil.

The total concentration of these hydrophobic compounds for forming emulsions is in the range of 0.1% to 20% by weight, preferably from 1% to 15% by weight, and more preferably from 2% to 10% by weight, calculated to the total of the composition.

Emulsions are illustrated in examples 5, 6, and 7.

**Surfactants**

The composition may further comprise surfactant(s), preferably a non-ionic and/or anionic and/or cationic and/or amphoteric/zwitterionic surfactant, in viewpoint of examples 4-8.

Suitable anionic surfactants are alkyl sulfate or preferably ethoxylated alkyl ether sulfate surfactant, and/or mixtures thereof with an alkyl chain length of $C_{10}$ to $C_{22}$.

Suitable surfactants are laureth sulfates, coceth sulfate, pareth sulfate, capryleth sulphate, myreth sulfate, oleth sulfate, deceth sulfate, trideceth sulfate, coco sulphate, $C_{10}$-$C_{16}$ alkyl sulphate, $C_{11}$-$C_{15}$ alkyl sulphate, $C_{12}$-$C_{18}$ alkyl sulphate, $C_{12}$-$C_{15}$ alkyl sulphate, $C_{12}$-$C_{16}$ alkyl sulphate, $C_{12}$-$C_{13}$ alkyl sulfate, lauryl sulphate, myrystyl sulphate, palm kernel sulphate, cetearyl sulfate, cetyl sulphate, decyl sulphate, oleyl sulphate, behenyl sulphate and/or their salts. All of the aforementioned anionic surfactants may or may not be ethoxylated at various degrees.

Further suitable anionic surfactants useful within the scope of the invention are $\alpha$-olefin sulfonates and/or their salts, and in particular alkali salts of sulfosuccinic acid semiesters, for example, the disodium salt of monooctyl sulfosuccinate and alkali salts of long-chain monoalkyl ethoxysulfosuccinates.

Suitable non-ionic surfactants are N-alkylpolyhydroxyalkylamide type surfactants according to the following general formula:

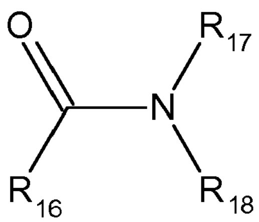

wherein $R_{16}$ is a linear or branched, saturated or unsaturated alkyl chain with $C_{11}$ to $C_{21}$, $R_{17}$ is linear or branched alkyl, or linear or branched hydroxyalkyl with $C_1$ to $C_4$, and $R_{18}$ is a linear or branched polyhydroxyalkyl chain with $C_3$ to $C_{12}$ and 3 to 10 hydroxyl groups.

Such compounds are disclosed in cosmetic compositions in WO96/27366 and their synthesis is disclosed in US1985424, US2016962, US2703798, and WO92/06984.

The preferred N-alkylpolyhydroxyalkylamide type surfactants have the following structure:

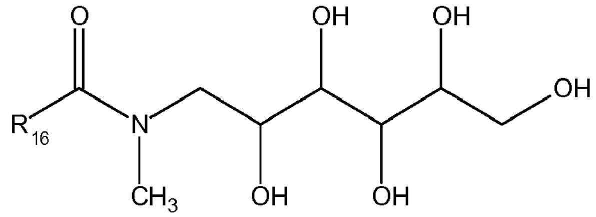

where $R_{16}$ has the same denotation as above for the general structure of N-alkylpolyhydroxyalkylamide type surfactants. The preferred surfactants as displayed above are known as N-methyl-N-acylglucamides.

The most preferred N-alkylpolyhydroxyalkylamide type surfactants are selected from lauroyl/myristoyl methyl glucamide and coco methyl glucamide.

Further suitable nonionic surfactants are alkyl polyglycosides according to the general structure:

$$R_{23}O(R_{24}O)_tZ_x$$

Wherein Z denotes a carbohydrate with $C_5$ to $C_6$, $R_{23}$ is an alkyl group with $C_8$ to $C_{18}$, $R_{24}$ is methyl, ethyl or propyl, t ranges from 0 to 10, and x ranges from 1 to 5. Suitable compounds according to this structure are $C_9$-$C_{11}$ alkylpolyglycoside, the structures disclosed in EP-A 70 074, and JP 2015-123019A.

The preferred compounds according to the structure of above are decyl glucoside, lauryl glucoside, and coco glucoside, and the most preferred one is decyl glucoside.

Suitable examples for non-ionic surfactants are fatty alcohol ethoxylates of the following general structure

$$R_{25}(OCH_2CH_2)_{n4}OH$$

wherein $R_{25}$ is straight or branched, saturated or unsaturated alkyl chain which may be synthetic or natural with a C chain length in the range of 8 to 40, preferably 9 to 30 and more preferably 9 to 24 and n4 is a number in the range of 5 to 40, preferably 9 to 30.

Non-limiting suitable examples of the fatty alcohol ethoxylates are $C_{9-11}$ Pareth-6, $C_{9-11}$ Pareth-8, $C_{9-15}$ Pareth-8, $C_{11-13}$ Pareth-9, $C_{11-13}$ Pareth-10, $C_{11-15}$ Pareth-5, $C_{11-15}$ Pareth-7, $C_{11-15}$ Pareth-9, $C_{11-15}$ Pareth-12, $C_{11-15}$ Pareth-15, $C_{11-15}$ Pareth-20, $C_{11-15}$ Pareth-30, $C_{11-15}$ Pareth-40, $C_{11-21}$ Pareth-10, $C_{12-13}$ Pareth-5, $C_{12-13}$ Pareth-6, $C_{12-13}$ Pareth-7, $C_{12-13}$ Pareth-9, $C_{12-13}$ Pareth-10, $C_{12-13}$ Pareth-15, $C_{12-13}$ Pareth-23, $C_{12-14}$ Pareth-5, $C_{12-14}$ Pareth-7, $C_{12-14}$ Pareth-9, $C_{12-14}$ Pareth-11, $C_{12-14}$ Pareth-12, $C_{12-15}$ Pareth-5, $C_{12-15}$ Pareth-7, $C_{12-15}$ Pareth-9, $C_{12-15}$ Pareth-10, $C_{12-15}$ Pareth-11, $C_{12-15}$ Pareth-12, $C_{12-16}$ Pareth-5, $C_{12-16}$ Pareth-7, $C_{12-16}$ Pareth-9, $C_{13-15}$ Pareth-21, $C_{14-15}$ Pareth-7, $C_{14-15}$ Pareth-8, $C_{14-15}$ Pareth-11, $C_{14-15}$ Pareth-12, $C_{14-15}$ Pareth-13, $C_{20-22}$ Pareth-30, $C_{20-40}$ Pareth-10, $C_{20-40}$ Pareth-24, $C_{20-40}$ Pareth-40, $C_{20-40}$ Pareth-95, $C_{22-24}$ Pareth-33, Beheneth-5, Beheneth-10, Beheneth-15, Beheneth-20, Beheneth-25, Beheneth-30, Ceteareth-5, Ceteareth-6, Ceteareth-7, Ceteareth-10, Ceteareth-11, Ceteareth-12, Ceteareth-15, Ceteareth-20, Ceteareth-25, Ceteareth-30, Ceteareth-35, Ceteareth-40, Laureth-5, Laureth-10, Laureth-15, Laureth-20, Laureth-25, Laureth-30, Laureth-40, Myreth-5, Myreth-10, Ceteth-5, Ceteth-10, Ceteth-15, Ceteth-20, Ceteth-25, Ceteth-30, Ceteth-40, Oleth-5, Oleth-10, Oleth-15, Oleth-20, Oleth-25, Oleth-30, Oleth-40, Steareth-5, Steareth-10, Steareth-15, Steareth-20, Steareth-25, Steareth-30, Steareth-35, and Steareth-40. They may also be present in the compositions as a mixture of more than one surfactant.

Further suitable nonionic surfactants are polypropylene glycol ethers of fatty alcohol according to general structure

$$R_{25}(OCH_2\text{-}CH_2\text{-}CH_2)_{n5}OH$$

wherein $R_{25}$ is straight or branched, saturated or unsaturated fatty alcohol which may be synthetic or natural with a C chain length in the range of 8 to 40, preferably 9 to 30 and more preferably 9 to 24 and n5 is a number in the range of 1 to 40, preferably 3 to 30.

Suitable non-limiting examples are PPG-3 Caprylyl ether, PPG-5 Caprylyl ether, PPG-10 Caprylyl ether, PPG-10 Cetyl ether, PPG-20 Cetyl ether, PPG-28 Cetyl ether, PPG-30 Cetyl ether, PPG-7 Lauryl ether, PPG-10 Lauryl ether, PPG-10 Oleyl ether, PPG-20 Oleyl ether, PPG-23 Oleyl ether, PPG-30 Oleyl ether, PPG-11 Stearyl ether and PPG-15 Stearyl ether.

Further suitable nonionic surfactants are polyethylene glycol fatty acid esters of the following general structure

$$R_{26}C(O)(OCH_2CH_2)_{n6}OH$$

wherein $R_{26}$ is straight or branched, saturated or unsaturated alkyl group which may be synthetic or natural with a C chain length in the range of 7 to 39, preferably 9 to 29 and more preferably 9 to 23 and n6 is a number in the range of 5 to 40, preferably 9 to 30.

Suitable non-limiting examples are PEG-8 Behenate, PEG-8 Caprate, PEG-8 Caprylate, PEG-5 Cocoate, PEG-8 Cocoate, PEG-9 Cocoate, PEG-10 Cocoate, PEG-15 Cocoate, PEG-6 Isopalmitate, PEG-6 Isostearate, PEG-8 Isostearate, PEG-9 Isostearate, PEG-10 Isostearate, PEG-12 Isostearate, PEG-20 Isostearate, PEG-30 Isostearate, PEG-40 Isostearate, PEG-6 Laurate, PEG-8 Laurate, PEG-9 Laurate, PEG-10 Laurate, PEG-12 Laurate, PEG-14 Laurate, PEG-20 Laurate, PEG-30 Laurate, PEG-8 Myristate, PEG-20 Myristate, PEG-5 Oleate, PEG-6 Oleate, PEG-7 Oleate, PEG-8 Oleate, PEG-9 Oleate, PEG-10 Oleate, PEG-11 Oleate, PEG-12 Oleate, PEG-15 Oleate, PEG-20 Oleate, PEG-30 Oleate, PEG-32 Oleate, PEG-6 Palmitate, PEG-18 Palmitate, PEG-20 Palmitate, PEG-5 Stearate, PEG-6 Stearate, PEG-7 Stearate, PEG-8 Stearate, PEG-9 Stearate, PEG-10 Stearate, PEG-12 Stearate, PEG-14 Stearate, PEG-15 Stearate, PEG-20 Stearate, PEG-25 Stearate, PEG-30 Stearate, PEG-35 Stearate and PEG-40 Stearate.

Further suitable nonionic surfactants are polypropylene glycol fatty acid esters of the following general structure

$$R_{27}C(O)(OCH_2\text{-}CH_2\text{-}CH_2)_{n8}OH$$

wherein $R_{27}$ is straight or branched, saturated or unsaturated alkyl group which may be synthetic or natural with a C chain length in the range of 7 to 39, preferably 9 to 29 and more preferably 9 to 23 and n8 is a number in the range of 1 to 40, preferably 9 to 30.

Suitable non-limiting examples are PPG-15 Isostearate, PPG-9 Laurate, PPG-26 Oleate and PPG-36 Oleate.

Further suitable nonionic surfactants are polyethylene glycol and polypropylene glycol ether of fatty alcohols of the following general structure

$$R_{28}(OCH_2\text{-}CH_2\text{-}CH_2)_{n9}(OCH_2CH_2)_{n10}\ OH$$

wherein $R_{28}$ is straight or branched, saturated or unsaturated alkyl group which may be synthetic or natural with a C chain length in the range of 7 to 39, preferably 9 to 29 and more preferably 9 to 23 and n9 and n10 may be the same or different and are a number in the range of 1 to 40.

Further suitable nonionic surfactants are ethoxylated vegetable oils. Well-known and commonly used examples are ethoxylated castor oil such as PEG-40 hydrogenated castor oil or and PEG-60 hydrogenated castor oil.

Suitable cationic surfactants have one or more positively charged group(s) at a pH 4 or less and are selected from cationic and/or cationizable and/or zwitterionic/amphoteric surfactants.

Suitable cationic surfactants are of quaternary ammonium structure according to the following general structure

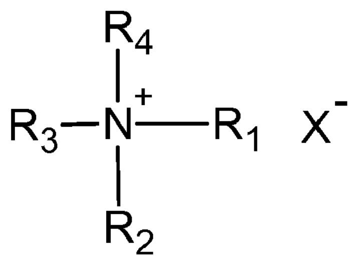

where $R_1$ is a saturated or unsaturated, branched or linear alkyl chain with $C_8$-$C_{22}$ or

$$R_5CONH(CH_2)_n$$

where $R_5$ is saturated or unsaturated, branched or linear alkyl chain with $C_7$-$C_{21}$ atoms and n has typical value of 1-4 or

$$R_6COO(CH_2)_n$$

where $R_6$ is saturated or unsaturated, branched or linear alkyl chain with $C_7$-$C_{21}$ atoms and n has typical value of 1-4, and

$R_2$ is unsaturated or saturated, branched or linear alkyl chain with $C_1$-$C_{22}$ atoms or

$$R_5CONH(CH_2)_n$$

or

$R_6COO(CH_2)_n$

where $R_5$, $R_6$ and n are same as above.

$R_3$ and $R_4$ have an alkyl chain with $C_1$ to $C_4$, and $X^-$ is typically chloride, bromide, or methosulfate.

Typical examples of those ingredients are cetyl trimethyl ammonium chloride, stearyl trimonium chloride, dipalmitoyl dimonium chloride, distearyl dimethyl ammonium chloride, stearamidopropyl trimethyl ammonium chloride, dioleoylethyl dimethyl ammonium methosulfate, dioleoylethyl hydroxyethylmonium methosulfate, behenyl trimethyl ammonium chloride, and/or their mixtures.

Suitable cationizable surfactants are surfactants which carry one or more chemical group(s) that is/are at least at some point non-ionic at pH above 4, but which is/are positively charged at a pH of 4 or less. Such groups are, for example, primary, secondary, and tertiary amino groups.

Further suitable cationizable surfactants are known as alkyl amido alkyl amine surfactants and are according to the following general structure

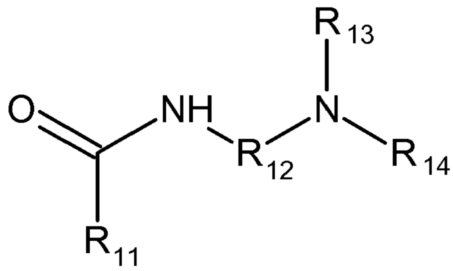

where $R_{11}$ is a saturated or unsaturated, straight or branched alkyl chain with $C_{11}$ to $C_{21}$, $R_{12}$ is a straight or branched alkyl chain with $C_1$ to $C_6$, $R_{13}$ and $R_{14}$ may be the same of different selected from H and straight or branched alkyl chain with $C_1$ to $C_4$.

Suitable compounds according to this definition are, for example, cocamidopropyl dimethylamine, stearamidopropyl dimethylamine, behenamidopropyl dimethylamine.

Further suitable surfactants having one or more positively charged group(s) at a pH below 7 are amphoteric/zwitterionic surfactants. Amphoteric/zwitterionic surfactants may be selected from compounds according to the general structure(s)

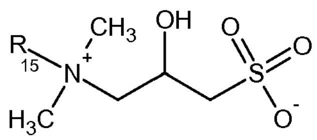

and/or

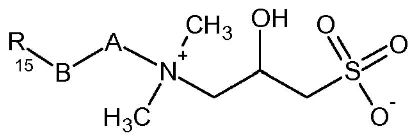

wherein $R_{15}$ is a straight or branched, saturated or unsaturated, substituted or unsubstituted alkyl chain with a carbon number of $C_{10}$ to $C_{22}$, preferably $R_{15}$ is a straight alkyl chain with a carbon number of $C_{10}$ to $C_{16}$, A is a straight alkyl chain with a carbon number of $C_1$ to $C_6$ or a branched alkyl chain with a carbon number of $C_3$ to $C_6$, preferably A is a linear alkyl chain with a carbon number of $C_3$, and B is an amide or an ester group.

Suitable compounds are known as hydroxysultaine surfactants, such as cocoamidopropyl hydroxysultaine, laurylamidopropyl hydroxysultaine, erucamidopropyl hydroxysultaine, lauryl hydroxysultaine, and cocoyl hydroxysultaine.

Further suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine.

It is to be noted that the skilled person can combine the surfactants from the classes of above to create suitable surfactant mixtures.

The preferred surfactant(s) having one or more positively charged group(s) at a pH of 4 or less of the composition according to the present invention is/are selected from quaternary ammonium surfactant(s), alkyl amido alkyl amine surfactant(s), amphoteric/zwitterionic surfactants, and/or their mixtures.

The total concentration of surfactants in the composition of the present invention is in the range of 0.05% to 25% by weight, preferably 0.1% to 15% by weight, more preferably in the range of 0.2% to 8% by weight, calculated to the total of the composition.

**Cationic polymers**

The composition of the present invention may comprise one or more cationic conditioning polymer(s) different from Polysilicone-29.

Suitable cationic polymers are those of best known with their INCI category name Polyquaternium.

Typical examples of those are Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium 49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86, and Polyquaternium 87.

It has further been found that especially those of cationic cellulose type polymers known as Polymer JR type from Amerchol such as Polyquaternium 10 or cationic galactomannans such as cationic guar gum known with trade name Jaguar from Rhône-Poulenc which are chemically for example guar hydroxypropyl trimonium chloride and cationic tara gum and its derivatives known with INCI name Caesalpinia spinosa hydroxypropyltrimonium chloride, are preferred ones. Furthermore, chitosan and chitin can also be included in the compositions as cationic natural polymers. In this context reference is also made to the cationic polymers disclosed in DE 25 21 960, DE 28 11 010, 30 44 738 and DE 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, cationic Caesalpinia spinosa gum derivatives, polyquaternium 6, polyquaternium 7, polyquaternium 10, polyquaternium 37, polyquaternium 67 and polyquaternium 70.

The cationic polymers also include the quaternized products of graft polymers from organopolysiloxanes and polyethyl oxazolines described in EP-A 524 612 and EP-A 640 643.

Compositions may comprise cationic polymer at a total concentration of 0.01 to 5%, preferably 0.02 to 4%, more preferably 0.05 to 3% and most preferably 0.1 to 2.5% by weight, calculated to the total of the composition.

This viewpoint is illustrated in example 5.

The following examples are to illustrate the invention, but not to limit it.

**EXAMPLES**

**Example 1**

The following compositions were prepared by conventional mixing techniques:

| **Ingredient** | **Inventive comp [% by weight]** | **Comparative comp A [% by weight]** | **Comparative comp B [% by weight]** | **Comparative comp C [% by weight]** |
|---|---|---|---|---|
| **Glyoxylic acid** | 3.7 | 4.0 | - | 3.7 |
| **Polysilicone-29** | 0.3 | - | 4.0 | - |
| **PEG-40/PPG-8 Methylaminopropyl/ Hydroxypropyl Dimethicone Copolymer** | - | - | - | 0.3 |

(continued)

| Ingredient | Inventive comp [% by weight] | Comparative comp A [% by weight] | Comparative comp B [% by weight] | Comparative comp C [% by weight] |
|---|---|---|---|---|
| **NaOH / HCl** | Ad pH 2.0 | | | |
| **Water** | Ad 100.0 | | | |

Indian frizzy hair streaks (25 cm long, 2 g per bundle) were purchased from International Hair Importers & Products Inc., Glendale, NY, USA. One hair streak was left untreated and kept for reference purposes. The hair streaks were washed with a commercially available shampoo under the tradname Goldwell Kerasilk Control Purifying Shampoo and then air-dried for 15 h. The volume of the hair streaks was measured with a machine rotating the hair streak, taking a photograph, and analyzing the area of the resulting hair cone ($V_0$). 2 g of the compositions from above were applied onto a hair streak and left on the streaks for 20 min at room temperature. The hair streaks were then blow dried and treated with a straightening iron having a surface temperature of 200°C (3 strokes per streak). The hair streaks were then shampooed again with the commercially available shampoo from above and allowed to air-dry for 15 h. The volume of the hair streaks was measured as described above ($V_1$). Each experiment with the compositions from above was run in triplicates.

The volume reduction was calculated according to the following formula:

$$V_{red} = \frac{V_1}{V_0} \cdot 100\%$$

The following results were obtained:

| Treatment | $V_{red}$ [%] | SD |
|---|---|---|
| **Inventive comp** | 36.7 | 2.3 |
| **Comparative comp A** | 41.2 | 1.2 |
| **Comparative comp B** | 45.1 | 2.9 |
| **Comparative comp C** | 43.2 | 1.6 |

As the results from above clearly show, the inventive composition delivered a higher straightening effect in comparison to the comparative compositions.

The keratin fibers treated with the inventive composition had the best cosmetic feel in comparison to the comparative compositions.

Moreover, 4 trained experts in the field of hair care confirmed independently of each other that the was no discernible color difference between the reference hair streak and the hair streak treated with the inventive composition whereas the hair streaks treated with the comparative compositions appeared lighter brownish than the reference hair streak.

**Example 2**

The following compositions were prepared by conventional mixing techniques:

| Ingredient | Inventive comp [% by weight] | | Comparative comp A [% by weight] | | Comparative comp B [% by weight] | |
|---|---|---|---|---|---|---|
| **Steps** | 1 | 2 | 1 | 2 | 1 | 2 |
| **Glyoxylic acid** | 3.7 | - | 4.0 | - | - | - |
| **Polysilicone-29** | - | 0.3 | - | - | 4.0 | - |

(continued)

| Ingredient | Inventive comp [% by weight] | | Comparative comp A [% by weight] | | Comparative comp B [% by weight] | |
|---|---|---|---|---|---|---|
| **Steps** | 1 | 2 | 1 | 2 | 1 | 2 |
| **PEG-40/PPG-8 Methylaminopropyl/ Hydroxypropyl Dimethicone Copolymer** | - | - | - | - | - | - |
| **NaOH / HCl** | Ad pH 2.0 | | | | | |
| **Water** | Ad 100.0 | | | | | |

Indian frizzy hair streaks (25 cm long, 2 g per bundle) were purchased from International Hair Importers & Products Inc., Glendale, NY, USA. One hair streak was left untreated and kept for reference purposes. The hair streaks were washed with a commercially available shampoo under the tradename Goldwell Kerasilk Control Purifying Shampoo and then air-dried for 15 h. The volume of the hair streaks was measured as disclosed in example 1 ($V_0$). 2 g of the compositions of step 1 from above were applied onto a hair streak and left on the streaks for 20 min at room temperature. The hair streaks were then rinsed thoroughly with lukewarm water, towel dried, and 2 g of the composition of step 2 was applied. The hair streaks were blow dried and treated with a straightening iron having a surface temperature of 200°C (3 strokes per streak). The hair streaks were then shampooed again with the commercially available shampoo from above and allowed to air-dry for 15 h. The volume of the hair streaks was measured as described above ($V_1$). Each experiment with the compositions from above was run in triplicates.

The volume reduction was calculated according to the following formula:

$$V_{red} = \frac{V_1}{V_0} \cdot 100\%$$

The following results were obtained:

| Treatment | $V_{red}$ [%] | SD |
|---|---|---|
| **Inventive comp** | 46.8 | 1.8 |
| **Comparative comp A** | 59.6 | 1.8 |
| **Comparative comp B** | 60.2 | 7.8 |

As the results from above clearly show, the inventive composition delivered a higher straightening effect in comparison to the comparative compositions. Moreover, the straightening performance of the inventive composition was similar to the one-step treatment of example 1.

The keratin fibers treated with the inventive composition had the best cosmetic feel in comparison to the comparative compositions.

Moreover, 4 trained experts in the field of hair care confirmed independently of each other that the was no discernible color difference between the reference hair streak and the hair streak treated with the inventive composition whereas the hair streaks treated with the comparative compositions appeared lighter brownish than the reference hair streak.

The following examples are within the scope of the present invention.

**Example 3**

| | **% by weight** |
|---|---|
| Glyoxylic acid | 1.0 |
| Polysilicone 29 | 0.1 |
| Ceteareth 3 | 0.1 |

(continued)

| | % by weight |
|---|---|
| Cetrimonium chloride | 1.0 |
| Dehydroxanthan gum | 0.5 |
| NaOH/HCl | ad pH 2.5 |
| Water | ad 100.0 |

The concentration of glyoxylic acid can be adjusted in this example to 3%, 5%, 10%, 15%, 20% by weight and all values within this range.

The pH may equally be adjusted in the range of 1.0 to 4.0.

**Example 4**

| | % by weight |
|---|---|
| Glyoxylic acid | 0.1 |
| Polysilicone 29 | 1.0 |
| Ceteareth 3 | 0.1 |
| Cetrimonium chloride | 1.0 |
| Dehydroxanthan gum | 0.5 |
| NaOH/HCl | ad pH 2.5 |
| Water | ad 100.0 |

The concentration of Polysilicone 29 can be adjusted in this example to 3%, 5%, 10%, by weight and all values within this range.

**Example 5**

| | % by weight |
|---|---|
| Glyoxylic acid | 3.0 |
| Polysilicone 29 | 0.1 |
| Cetearyl alcohol | 5.0 |
| Isopropyl palmitate | 1.0 |
| PEG-40 hydrogenated castor oil | 1.0 |
| Cetrimonium chloride | 1.0 |
| Behenamidopropyl dimethylamine | 0.5 |
| Polyquaternium 10 | 0.2 |
| Fragrance | 0.2 |
| Preservatives | 0.1 |
| NaOH/HCl | ad pH 3.0 |
| Water | ad 100.0 |

The concentration of glyoxylic acid can be adjusted in this example to 3%, 5%, 10%, 15%, 20% by weight and all values within this range.

**Example 6**

**Step 1**

| | % by weight |
|---|---|
| Glyoxylic acid | 3.0 |
| Cetearyl alcohol | 5.0 |
| Isopropyl palmitate | 1.0 |
| PEG-40 hydrogenated castor oil | 1.0 |
| Cetrimonium chloride | 1.0 |
| Behenamidopropyl dimethylamine | 0.5 |
| Fragrance | 0.2 |
| Preservatives | 0.1 |
| NaOH/HCl | ad pH 3.0 |
| Water | ad 100.0 |

The concentration of glyoxylic acid can be adjusted in this example to 3%, 5%, 10%, 15%, 20% by weight and all values within this range.

**Step 2**

| | % by weight |
|---|---|
| Polysilicone 29 | 0.5 |
| Cetearyl alcohol | 5.0 |
| Cetrimonium chloride | 2.0 |
| Behenamidopropyl dimethylamine | 0.5 |
| Fragrance | 0.2 |
| Preservatives | 0.1 |
| NaOH/HCl | ad pH 3.0 |
| Water | ad 100.0 |

The concentration of Polysilicone 29 can be adjusted in this example to 0.1% 3%, 5%, 10%, by weight and all values within this range.

The application of the compositions follows the procedure of example 2.

**Example 7**

**Step 1**

| | % by weight |
|---|---|
| Glyoxylic acid | 3.0 |
| PEG-40 hydrogenated castor oil | 0.2 |
| Xanthan gum | 0.5 |
| NaOH/HCl | ad pH 3.0 |
| Water | ad 100.0 |

The concentration of glyoxylic acid can be adjusted in this example to 3%, 5%, 10%, 15%, 20% by weight and all values within this range.

**Step 2**

| | % by weight |
|---|---|
| Polysilicone 29 | 0.5 |
| Cetearyl alcohol | 5.0 |
| Cetrimonium chloride | 2.0 |
| Behenamidopropyl dimethylamine | 0.5 |
| Fragrance | 0.2 |
| Preservatives | 0.1 |
| NaOH/HCl | ad pH 3.0 |
| Water | ad 100.0 |

The concentration of Polysilicone 29 can be adjusted in this example to 0.1% 3%, 5%, 10%, by weight and all values within this range.

The application of the compositions follows the procedure of example 2.

**Example 8**

**Composition A**

The composition of step 1 of example 7.

**Composition B**

| | % by weight |
|---|---|
| Polysilicone 29 | 0.5 |
| Dehydroxanthan gum | 0.25 |
| NaOH/HCl | ad pH 8.0 |
| Water | ad 100.0 |

The concentration of Polysilicone 29 can be adjusted in this example to 0.1% 3%, 5%, 10%, by weight and all values within this range.

The pH of composition B may be adjusted to 2.0, 5.0, or 10.0.

The compositions A and B are mixed prior to application in a weight ratio of 1:1 and are then applied onto keratin fibers.

**Claims**

1. A composition for straightening of keratin fibers, preferably human keratin fibers, more preferably human hair, having a pH of 4 or less and comprising

    - glyoxylic acid and/or its hydrate(s) and/or its salt(s),
    - Polysilicone-29.

2. The composition according to claim 1 **characterized in that** the total concentration of glyoxylic acid and/or its hydrate(s) and/or its salt(s) is in the range of 0.1% to 20% by weight, preferably in the range of 0.2% to 10% by weight, more preferably in the range of 0.25% to 5% by weight, further more preferably in the range of 0.35% to 5% by weight, calculated to the total of the composition.

3. The composition according to claims 1 and/or 2 **characterized in that** the total concentration of Polysilicone-29 is in the range of 0.01% to 10% by weight, preferably in the range of 0.02% to 8% by weight, more preferably in the range of 0.05% to 6% by weight, further more preferably in the range of 0.1% to 2% by weight, calculated to the total of the composition.

4. The composition according to any of the preceding claims **characterized in that** the weight ratio of glyoxylic acid and/or its hydrate(s) and/or its salt(s) to Polysilicone-29 is in the range of 10 to 0.1.

5. The composition according to any of the preceding claims **characterized in that** the composition is a thickened gel and comprises a thickening agent, preferably a thickening polymer, more preferably an associative and/or non-associative thickening polymer, further more preferably a carbohydrate-based non-associative thickening polymer, still further more preferably it is selected from xanthan gum and/or dehydroxanthan gum.

6. The composition according to any of the preceding claims **characterized in that** the composition is an emulsion and comprises one or more hydrophobic compound(s), preferably selected from $C_{12}$ to $C_{22}$ fatty alcohols, fatty acid esters having a $C_{12}$ to $C_{22}$ fatty acid esterified with a linear or branched, saturated or unsaturated $C_3$ to $C_{18}$ fatty alcohol, natural and/or vegetable oils, hydrocarbon-based compounds, and/or their mixtures.

7. The composition according to any of the preceding claims **characterized in that** it further comprises a surfactant, preferably an anionic and/or non-ionic and/or cationic and/or amphoteric/zwitterionic surfactant.

8. The composition according to any of the preceding claims **characterized in that** the total concentration of surfactants is in the range of 0.05% to 25% by weight, preferably 0.1% to 15% by weight, more preferably in the range of 0.2% to 8% by weight, calculated to the total of the composition.

9. The composition according to any of the preceding claims **characterized in that** it comprises one or more cationic conditioning polymer(s) different from Polysilicone-29.

10. The composition according to any of the preceding claims **characterized in that** the total concentration of cationic polymers is in the range of 0.01 to 5%, preferably 0.02 to 4%, more preferably 0.05 to 3% and most preferably 0.1 to 2.5% by weight, calculated to the total of the composition.

11. A two-part composition comprising the parts A and B, which are provided for separate storage and for mixing directly prior to the application onto the keratin fibers, wherein part A comprises glyoxylic acid and/or its hydrate(s) and/or its salt(s) having a pH of less than 4, and part B comprises Polysilicone-29 having a pH in the range of 2 to 10.

12. A process for semi-permanent straightening of keratin fibers, preferably human keratin fibers, more preferably human hair, **characterized in that** it comprises the steps of:

a) optionally washing the keratin fibers and/or drying the keratin fibers,
b) applying the composition as defined in claims 1 to 10 and/or the composition as defined in claim 11 onto keratin fibers,
c) leaving the composition onto the keratin fibers for 1 min to 120 min, preferably for a time period in the range of 2 min to 60 min, more preferably in the range of 2 min to 30 min, further more preferably in the range of 2 min to 10 min,
d) optionally rinsing-off the keratin fibers,
e) drying the keratin fibers,
f) treating the keratin fibers with a straightening iron having a surface temperature of 180°C $\pm$ 50°C, preferably in the range of 170°C to 200°C,
g) optionally rinsing-off and/or washing the keratin fibers and/or drying the keratin fibers.

13. The process according to claim 12 **characterized in that** the steps a) to g) are repeated with a time period of 8 h and 72 h between each full process run.

14. A process for semi-permanent straightening of keratin fibers, preferably human keratin fibers, more preferably human hair **characterized in that** it comprises the steps of:

a) optionally washing the keratin fibers and/or drying the keratin fibers,
b) applying a composition having a pH of 4 or less and comprising glyoxylic acid and/or its hydrate(s) and/or its salt(s) as defined in claim 11,
c) leaving the composition onto the keratin fibers for 1 min to 120 min, preferably for a time period in the range of 2 min to 60 min, more preferably in the range of 2 min to 30 min, further more preferably in the range of 2 min to 10 min,

d) rinsing-off the keratin fibers,
e) applying a composition having a pH of 2 to 10 and comprising Polysilicone-29,
f) optionally rinsing-off the keratin fibers,
g) drying the keratin fibers,
h) treating the keratin fibers with a straightening iron having a surface temperature of 180°C ± 50°C, preferably in the range of 170°C to 200°C,
i) optionally rinsing-off and/or washing the keratin fibers and/or drying the keratin fibers.

**15.** A kit-of-parts comprising the composition as defined in the claims 1 to 10 and/or as defined in claim 11 and a straightening tool for keratin fibers, preferably a hair straightening iron.

Europäisches Patentamt
European Patent Office
Office européen des brevets

# EUROPEAN SEARCH REPORT

Application Number
EP 18 20 9099

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/131469 A1 (KAO CORP [JP]) 4 September 2014 (2014-09-04) * pages 5-13; claims 1-25 * * pages 25, 28; examples 3-11 * ----- | 1-15 | INV. A61K8/365 A61Q5/06 A61K8/898 |
| Y | Anonymous: "Silsoft CLX-E conditioning agent Marketing Bulletin", Momentive , 2015, pages 1-12, XP002788416, Retrieved from the Internet: URL:www.momentive.com [retrieved on 2019-01-29] * pages 5-7 * ----- | 1-15 | |
| Y | US 2015/258007 A1 (BIATO CAMILA [BR] ET AL) 17 September 2015 (2015-09-17) * claims 1-6; example 1 * ----- | 1-15 | |
| Y,D | FR 2 997 845 A1 (OREAL [FR]) 16 May 2014 (2014-05-16) * claims 1-13; example 1 * ----- | 14 | TECHNICAL FIELDS SEARCHED (IPC) A61K A61Q |

The present search report has been drawn up for all claims

4

| Place of search | Date of completion of the search | Examiner |
|---|---|---|
| Munich | 30 January 2019 | Szarek, Sophie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 18 20 9099

This annex lists the patent family members relating to the patent documents cited in the above-mentioned European search report. The members are as contained in the European Patent Office EDP file on
The European Patent Office is in no way liable for these particulars which are merely given for the purpose of information.

30-01-2019

| Patent document cited in search report | Publication date | Patent family member(s) | Publication date |
|---|---|---|---|
| WO 2014131469 A1 | 04-09-2014 | AU 2013380233 A1 | 27-08-2015 |
| | | BR 112015020478 A2 | 18-07-2017 |
| | | CN 105188649 A | 23-12-2015 |
| | | EP 2961375 A1 | 06-01-2016 |
| | | HK 1217437 A1 | 13-01-2017 |
| | | JP 6276785 B2 | 07-02-2018 |
| | | JP 2016510008 A | 04-04-2016 |
| | | RU 2015140971 A | 31-03-2017 |
| | | SG 11201506756S A | 29-09-2015 |
| | | TW 201436809 A | 01-10-2014 |
| | | US 2016000671 A1 | 07-01-2016 |
| | | WO 2014131469 A1 | 04-09-2014 |
| US 2015258007 A1 | 17-09-2015 | BR 112015010525 A2 | 11-07-2017 |
| | | DE 202013011678 U1 | 14-02-2014 |
| | | EP 2916802 A1 | 16-09-2015 |
| | | FR 2997847 A1 | 16-05-2014 |
| | | US 2015258007 A1 | 17-09-2015 |
| | | WO 2014072478 A1 | 15-05-2014 |
| FR 2997845 A1 | 16-05-2014 | EP 2916806 A1 | 16-09-2015 |
| | | FR 2997845 A1 | 16-05-2014 |
| | | US 2015305469 A1 | 29-10-2015 |
| | | WO 2014072645 A1 | 15-05-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011104282 A
- WO 2012010351 A
- WO 2014067702 A
- FR 2997845
- FR 3044903
- FR 2997847 A
- WO 9627366 A
- US 1985424 A
- US 2016962 A
- US 2703798 A
- WO 9206984 A
- EP 70074 A
- JP 2015123019 A
- DE 2521960
- DE 28110103044738
- DE 3217059
- EP 337354 A
- EP 524612 A
- EP 640643 A